# FASCICULE DE BREVET EUROPEEN

(11) **EP 3 054 873 B1**
(45) Date de publication et mention de la délivrance du brevet: **25.10.2017**
(21) Numéro de dépôt: 14790230.8
(22) Date de dépôt: 07.10.2014
(51) Int. Cl.: A61B 17/70

(54) **DISPOSITIF IMPLANTABLE D'ARTHRODÈSE VERTÉBRALE POUR LA FUSION DE DEUX VERTÈBRES SUS ET SOUS JACENTES**
IMPLANTIERBARE VERTEBRALE ARTHRODESEVORRICHTUNG ZUR FUSION ZWEIER DARÜBER UND DARUNTER LIEGENDER WIRBEL
IMPLANTABLE VERTEBRAL ARTHRODESIS DEVICE FOR FUSING TWO OVERLYING AND UNDERLYING VERTEBRAE

(30) Priorité: 07.10.2013 FR 1359691
(43) Date de publication de la demande: 17.08.2016
(73) Titulaire: Cousin Biotech, 59117 Wervicq Sud (FR)
(72) Inventeur: DENEUVILLERS, Guy, F-62155 Merlimont (FR)
(74) Mandataire: Balesta, Pierre
(86) Numéro de dépôt international: PCT/FR2014/052543
(87) Numéro de publication internationale: WO 2015/052431

(56) Documents cités:
- WO-A1-2005/044118
- WO-A1-2013/123497
- US-A1- 2007 191 837
- US-A1- 2012 215 262
- US-B1- 8 292 923

## Description

### Arrière-plan de l'invention

La présente invention concerne le domaine technique des dispositifs implantables d'arthrodèse vertébrale pour la fusion de deux vertèbres sus et sous-jacentes.

On comprend par arthrodèse toute fusion d'une articulation. Ces dispositifs implantables ont pour fonction de répartir les charges et surcharges entre deux vertèbres adjacentes (désignées ici vertèbres sus et sous-jacentes) créées par la dégénérescence discale (dans le cas notamment d'une hernie discale). Par exemple, dans le cas d'une discopathie dégénérative, une contrainte ou une lésion sur un disque peut entraîner un déchirement ou la formation d'une protubérance, entraînant éventuellement un contact et la compression d'une racine nerveuse rachidienne. Un contact sur le nerf peut entraîner une lombalgie intermittente.

Une méthode de traitement de ce type de pathologie consiste à immobiliser les vertèbres sus et sous-jacentes par une fusion osseuse tout en rétablissant la distance inter-épineuse desdites vertèbres et leur angle de lordose.

Les dispositifs implantables utilisés le plus couramment dans cette méthode de traitement chirurgicale comprennent deux tiges reliées aux vertèbres sus et sous-jacentes par des vis positionnées dans les pédicules, vertèbres auxquelles on applique un greffon d'ostéosynthèse positionné sur les parties externes des vis et des tiges.

Ce type de dispositif implantable impose une ouverture du champ opératoire importante entraînant une nécrose des tissus environnants manipulés sur le patient pour accéder aux pédicules et mettre en place les vis ainsi que le greffon.

De plus, les points de fixation sur les pédicules, à l'aide des vis pédiculaires, sont éloignés des corps vertébraux sur lesquels les charges les plus importantes sont exercées. Cette disposition génère un bras de levier important et ainsi un report de la charge appliquée initialement sur les vis pédiculaires pouvant entraîner une rupture de ces dernières. Pour remédier à cet inconvénient, selon l'état de dégénérescence du disque, ces dispositifs comprennent également une ou deux cage(s) inter-somatique(s), insérée(s) par la voie postérieure, latérale ou antérieure, ce qui augmente le temps de l'intervention chirurgicale et donc les risques pour le patient.

Il a été observé que le greffon osseux disposé dans ces cages, réduit de volume après son implantation, et ce notamment d'environ 20%. Or, il est nécessaire que le greffon osseux soit stimulé mécaniquement par les corps vertébraux entre lesquels il est en contact, afin que l'arthrodèse puisse se développer correctement. Dans le cas d'une cage inter-somatique rigide, il est ainsi nécessaire de remplir cette dernière de greffon, en sorte que le greffon osseux dépasse des extrémités de la cage. Cette dernière disposition peut compliquer davantage le réglage de la lordose et de la distance intervertébrale.

WO 2013/123497 a pour objet un dispositif de fusion inter-épineux comprenant une portion de corps centrale qui est définie entre des première et seconde parois opposées et inférieures, une paroi arrière et des premier et second bords supérieurs. La portion de corps centrale, ouverte sur sa partie postérieure, ne peut recevoir qu'un greffon sous forme de bloc solide.

US 2012/0215262 a pour objet un dispositif d'ostéosynthèse destiné à être placé entre les apophyses épineuses. Ce dispositif ne permet pas de réaliser une fusion inter-lamaire.

US 8.292.923 B1 a pour objet un implant destiné à être placé entre les apophyses épineuses. Cet implant ne fournit pas également d'appui inter-lamaire et ne permet pas de réaliser une fusion inter-lamaire. En outre, il n'est pas agencé pour recevoir au moins en partie les apophyses épineuses. En effet, la cavité centrale n'est de quelques millimètres et vient juste en contact superficielle avec les épineuses.

Il existe un besoin pour un dispositif d'arthrodèse vertébrale pour la fusion de deux vertèbres adjacentes, en particulier au niveau lombaire, notamment en traitement d'une discopathie dégénérative ou accidentelle, ne nécessitant pas l'emploi de vis et de tiges afin de réduire le champ opératoire sur le patient, et permettant de diminuer la nécrose des tissus environnants sur le site d'implantation.

Il existe également un besoin pour un dispositif d'arthrodèse vertébral permettant de régler la distance intervertébral et l'angle de la lordose entre les vertèbres sus et sous-jacentes du niveau vertébral à corriger.

### Objet et Résumé de l'invention

La présente invention a pour objet un dispositif implantable d'arthrodèse vertébrale pour la fusion de deux vertèbres sus et sous-jacentes palliant les problèmes précités, comprenant de manière avantageuse :
a. une partie antérieure comprenant une zone supérieure d'appui apte à recevoir la partie inférieure de la lame supérieure de la vertèbre sus-jacente et une zone inférieure d'appui apte à recevoir la partie supérieure de la lame inférieure de la vertèbre sous-jacente, les zones supérieure et inférieure d'appui étant distantes d'une hauteur h minimum pour le maintien d'un écart intervertébral ;
b. des moyens de retenue rigides agencés par rapport à la partie antérieure en sorte de bloquer la migration de ladite partie antérieure vers le canal rachidien ;
c. une partie postérieure, en liaison avec ladite partie antérieure, comprenant un logement principal ayant des première et seconde ouvertures au regard l'une de l'autre et aptes à recevoir en partie les apophyses épineuses respectivement des vertèbres sus et sous-jacentes ;
d. un élément de cloisonnement rigide disposé entre la partie antérieure et la partie postérieure qui délimite, au moins en partie avec la partie antérieure, un logement auxiliaire ayant des première et seconde ouvertures au regard l'une de l'autre, et agencé en sorte de recevoir en partie les lames desdites vertèbres sus-jacente et sous-jacente ;
e. un matériau d'ostéosynthèse granuleux disposé dans ledit logement principal et ledit logement auxiliaire.

On comprend par arthrodèse vertébrale, toute arthrodèse réalisée sur les vertèbres cervicales ou les vertèbres dorsales ou thoraciques ou les vertèbres lombaires ou encore les vertèbres du sacrum, de préférence sur les vertèbres lombaires.

On comprend par partie postérieure en liaison avec la partie antérieure, que la partie postérieure est solidaire directement de la partie antérieure ou par l'intermédiaire d'une ou plusieurs pièces différentes de la partie antérieure.

De préférence, on entend par élément rigide, en particulier désignant les moyens de retenue, éventuellement la partie antérieure et/ou la partie postérieure, ou encore l'élément de cloisonnement défini ci-après, tout élément suffisamment résistant à la déformation en fonctionnement sous l'application de charges générées par les niveaux vertébraux quelle que soit la position des vertèbres, notamment afin de maintenir ses dimensions pré-implantatoires une fois implanté, et ce à plus ou moins 5% près.

De préférence, les moyens de retenue, éventuellement la partie postérieure et/ou la partie antérieure et/ou l'élément de cloisonnement défini ci-après, sont choisis parmi les matériaux suivants, seul ou en combinaison : le polyétherétherkétone (PEEK) ou ses dérivés tel que le polyétherkétone (PEK), le titane, le polyéthylène téréphtalate (PET), le carbone, l'hydroxyapatite, l'os humain ou animal taillé.

Le matériau d'ostéosynthèse granuleux peut être de l'os humain et/ou animal et/ou artificiel.

S'agissant d'os humain et/ou d'os animal, le matériau d'ostéosynthèse granuleux peut comprendre des copeaux ou des compresses cortico-spongieuses (pouvant contenir de l'os cortical et/ou de l'os trabéculaire), des dérivés osseux calcifiés ou décalcifiés.

S'agissant d'os artificiel, le matériau d'ostéosynthèse granuleux peut être des phosphates, des sulfates ou des carbonates de tricalcium, ou de l'hydroxyapatite, ou du ciment osseux, tels que des céramiques ou des ciments osseux de polyméthacrylate de méthyle.

La partie antérieure fournit au dispositif implantable un appui le plus éloigné possible des apophyses épineuses permettant ainsi d'équilibrer ce dernier.

La combinaison du maintien d'un écart intervertébral via la partie antérieure avec une partie postérieure comprenant un logement dans lequel est disposé un matériau d'ostéosynthèse permet de réaliser une fusion osseuse au niveau des apophyses épineuses, limitant ainsi l'ouverture du champ opératoire sur le patient.

Les apophyses épineuses supérieure et inférieure activent mécaniquement le matériau d'ostéosynthèse granuleux disposé dans le logement principal en passant à travers les première et seconde ouvertures débouchant dans ledit logement principal.

Avantageusement, en sus d'une fusion osseuse inter-épineuses, le dispositif implantable selon l'invention permet de réaliser également une fusion osseuse inter-lamaires. Cette disposition améliore la fusion du niveau vertébral à corriger.

Le dispositif implantable selon l'invention peut être utilisé seul sans moyen de solidarisation postérieur des apophyses épineuses supérieure et inférieure desdites vertèbres sus et sous-jacentes entre-elles, si le ligament inter-épineux est laissé en place lors de l'intervention chirurgicale.

Si le ligament inter-épineux est ôté, ou selon le souhait du praticien, le dispositif implantable selon l'invention est combiné avec un moyen de solidarisation postérieur desdites apophyses épineuses supérieure et inférieure entre-elles. Ce moyen de solidarisation postérieur peut comprendre un élément longiligne ayant des première et seconde extrémités ainsi que des moyens de solidarisation, de préférence amovibles, desdites extrémités entre-elles. Ledit élément longiligne est de préférence disposé autour des apophyses épineuses supérieure et inférieure du niveau vertébral à corriger.

La partie postérieure peut être rigide ou dans un matériau pliable. Dans ce dernier cas, la partie postérieure est de préférence combinée avec un élément longiligne pourvu à ses extrémités de moyens de solidarisation desdites extrémités, en sorte d'exercer une action inverse à celle exercée par les apophyses épineuses sur la partie postérieure dans un matériau pliable rempli de matériau d'ostéosynthèse en position de lordose. Ledit élément longiligne est de préférence disposé autour des apophyses épineuses et donc autour de la partie postérieure du dispositif implantable selon l'invention.

La partie antérieure peut être rigide, telle que définie ci-dessous, ou dans un matériau pliable, de préférence dans une bande pliable telle que définie dans le présent texte. La bande pliable est de préférence pourvue de première et seconde extrémités solidarisées aux moyens de retenue et/ou à la partie postérieure et/ou audit élément de cloisonnement (défini ci-après).

Avantageusement, la bande pliable définie avec ledit élément de cloisonnement et/ou lesdits moyens de retenue et/ou ladite partie postérieure, un logement auxiliaire apte à recevoir un matériau d'ostéosynthèse granuleux. Cette disposition favorise une fusion inter-lamaires.

De préférence, l'élément de cloisonnement est disposé dans un plan sensiblement parallèle au plan dans lequel la partie antérieure est disposée.

La disposition d'un élément de cloisonnement permet de redonner un écart anatomique intervertébral et de redonner de la lordose.

Cet élément de cloisonnement permet de conférer au dispositif implantable selon l'invention trois points d'appui au niveau inter-lamaires en combinaison avec la partie antérieure: deux points d'appui sur la partie antérieure et un point d'appui sur l'élément de cloisonnement. Cette disposition permet ainsi au dispositif implantable selon l'invention d'éviter à la partie antérieure de basculer en arrière sur les apophyses épineuses, et donc améliore la stabilité du dispositif.

Dans une variante de réalisation, le logement principal est dans un matériau pliable, de préférence dans une bande pliable dont la première extrémité et la seconde extrémité sont solidarisées respectivement au premier bord latéral et au second bord latéral de ladite partie antérieure et/ou des moyens de retenue et/ou de l'élément de cloisonnement.

Avantageusement, le logement principal, étant dans un matériau pliable donc déformable, est apte à subir des micromouvements en compression ou en lordose qui vont stimuler le matériau d'ostéosynthèse granuleux et faciliter la fusion osseuse.

Avantageusement, lorsque le dispositif implantable comprend un élément de cloisonnement rigide, le matériau pliable est de préférence en liaison avec ledit élément de cloisonnement, soit directement, soit par l'intermédiaire des moyens de retenue et/ou de la partie antérieure.

Dans ce dernier cas, le logement principal a une forme en C selon sa section transversale dont les extrémités sont formées par les première et seconde extrémités de la bande pliable.

L'élément de cloisonnement améliore la tenue du logement principal et évite que ce dernier ait tendance à s'affaisser lorsqu'il est rempli d'un matériau d'ostéosynthèse granuleux.

Les première et seconde extrémités de la bande pliable peuvent être collées aux premier et second bords latéraux de la partie antérieure et/ou des moyens de retenue et/ou dudit élément de cloisonnement ou encore enroulées sur elles-mêmes dans deux fourreaux disposés sur les moyens de retenue et/ou l'élément de cloisonnement.

La bande pliable peut être dans au moins un matériau polymère biocompatible flexible, et peut être non résorbable, c'est-à-dire qu'elle reste de façon permanente dans le corps, ou semi-résorbable, c'est-à-dire que seulement une partie de la bande pliable reste de façon permanente dans le corps. La bande pliable peut être par exemple un film en silicone, ou dans un matériau textile, tel qu'un non-tissé, un tricot, une tresse ou un tissu. Ledit matériau textile peut être éventuellement revêtu selon sa face interne et/ou sa face externe d'un revêtement partiellement ou totalement résorbable ou non résorbable, par exemple en silicone et/ou en acide polylactique de forme L ou D (PLLA ou PDLA), ou dans un copolymère d'acide lactique ou glycolique (PLGA).

Le matériau textile est de préférence dans un ou plusieurs matériaux synthétiques, et peut être en polypropylène, en polyéthylène téréphtalate (PET), en polyamide tel que le PA6-6, en polytétrafluoroéthylène (PTFE), dans un mélange polypropylène/PTFE, ou leur mélange.

Dans une variante de réalisation, les moyens de retenue sont supportés par ledit élément de cloisonnement ou la partie postérieure.

Dans une variante de réalisation, la partie antérieure comprend des premier et second bords latéraux en liaison avec les premier et second bords latéraux de l'élément de cloisonnement respectivement par l'intermédiaire de première et seconde parties latérales.

De préférence, les première et seconde extrémités de la bande pliable du logement principal sont solidarisées, par exemple par collage, respectivement auxdites première et seconde parties latérales.
Dans une sous-variante, les première et seconde parties latérales divergent vers la partie antérieure.

Cette disposition permet d'optimiser le volume du logement auxiliaire délimité, au moins en partie, entre la partie antérieure et l'élément de cloisonnement afin que la quantité de matériau d'ostéosynthèse disposée dans ledit logement auxiliaire soit la plus importante possible. La surface de la fusion osseuse, en particulier inter-lamaires, est ainsi optimisée.

Dans une variante de réalisation, l'élément de cloisonnement, et éventuellement les moyens de retenue, a ou ont une forme générale de U ou de H.

Cette forme favorise la réception des lames et des apophyses épineuses dans le logement principal, et éventuellement dans le logement auxiliaire. Cette forme améliore également la quantité de matériaux d'ostéosynthèse granuleux qui peut être reçue dans le logement principal, et éventuellement dans le logement auxiliaire, ce qui favorise la qualité de la fusion osseuse.

Cette disposition permet également de donner un axe de pivotement à l'apophyse épineuse de la vertèbre sus-jacente, et éventuellement de recevoir l'apophyse épineuse de la vertèbre sus-jacente sans forcément la tailler.

Dans une variante de réalisation, la partie postérieure, en particulier la paroi arrière, comprend une paroi ayant un premier creux supérieur, notamment en forme de U, et éventuellement un second creux inférieur, notamment en forme de U. De préférence, la partie postérieure comprend une paroi en forme de H.

La partie postérieure ainsi agencée peut recevoir au moins en partie les apophyses épineuses sans qu'il ne soit nécessaire d'entailler ces dernières.

Dans une variante de réalisation, la zone supérieure d'appui de la partie antérieure a une forme convexe ; de préférence la zone inférieure d'appui de la partie antérieure a une forme concave.

Cette disposition permet à la partie antérieure de s'adapter à la forme anatomique des lames des vertèbres sus et sous-jacentes et améliore ainsi l'ergonomie dudit dispositif.

Dans une variante de réalisation, la face antérieure de la partie antérieure est concave.

Cette disposition permet d'éloigner la face antérieure de la partie antérieure de la dure-mère, et évite de comprimer le ligament jaune.

Dans une variante de réalisation, la partie antérieure est inclinée vers le logement principal.

La partie antérieure forme de préférence un angle α avec le plan d'appui (P) sur lequel repose le dispositif implantable selon l'invention.

De préférence, l'angle α est inférieur à 90°, encore de préférence compris entre 45° et 90°, plus particulièrement compris entre 45° et 75°.

Cette disposition favorise le réglage de la lordose des vertèbres sus et sous-jacentes.

Cette disposition améliore également l'ergonomie du dispositif en ce qu'il s'adapte mieux à la disposition des vertèbres sus et sous-jacentes qui sont naturellement en quinconce.

Dans une variante de réalisation, la zone inférieure d'appui de la partie antérieure comprend des premier et second pieds inclinés vers le logement principal.

Ces pieds ont pour fonction d'améliorer la stabilité de l'appui sur les lames de la vertèbre sous-jacente.

Dans une variante de réalisation, les moyens de retenue comprennent au moins une première projection supérieure et au moins une première projection inférieure, qui s'étendent du premier et/ou du second bord(s) latéra(l)(ux) dudit élément de cloisonnement, et sont pourvues à leurs extrémités d'orifices pour le passage de vis.

De préférence, lesdites vis combinées avec lesdites projections forment un moyen de solidarisation postérieur des apophyses épineuses supérieure et inférieure entre-elles.

Cette disposition permet de réduire le champ opératoire sur le patient et limite ainsi l'étendue des tissus nécrosés. Il n'est en effet pas nécessaire d'utiliser des vis et des tiges solidarisées aux pédicules qui sont éloignées anatomiquement des apophyses épineuses.

Dans une variante de réalisation, la bande pliable est un panneau textile, éventuellement partiellement revêtu d'un revêtement, ou dans un film dans au moins un matériau polymère.

Ledit revêtement peut être continu, telle une couche, ou discontinu, notamment disposé sous forme de points, de lignes ou de motifs géométriques tels que des losanges par exemple.

Dans une variante de réalisation, la partie antérieure, les moyens de retenue, éventuellement la partie postérieure et/ou l'élément de cloisonnement, sont une pièce, de préférence moulée.

Dans une variante de réalisation, la partie antérieure, les moyens de retenue, éventuellement la partie postérieure et/ou l'élément de cloisonnement, sont dans un matériau d'ostéosynthèse tel que défini ci-dessus, notamment non granuleux, par exemple en hydroxyapatite moulée.

Cette disposition améliore encore la surface de la fusion osseuse que ce soit au niveau inter-lamaires comme au niveau inter-épineux.

La présente invention a pour objet, selon un second aspect, un ensemble comprenant un dispositif implantable selon l'une quelconque des variantes de réalisation précédentes, et un moyen de solidarisation postérieur des apophyses épineuses supérieure et inférieure entre-elles.

De préférence, ledit moyen de solidarisation postérieur comprend un élément longiligne pourvu de première et de seconde extrémités, et comprend un moyen de solidarisation, de préférence amovible, desdites extrémités entre elles.

Un tel moyen de solidarisation postérieur peut être le dispositif implantable décrit dans FR 2.961.687 A1.

### Description détaillée des dessins

La présente invention sera mieux comprise à la lecture des trois exemples de réalisation suivants cités de manière non limitative, et décrits en référence avec les figures suivantes, annexées à la présente, dans lesquelles :
- les figures **1A** et **1B** sont des représentations schématiques et en perspective d'un premier exemple de dispositif implantable selon l'invention ;
- la figure **2** est une représentation schématique et vue de dessous du premier exemple de dispositif implantable selon l'invention représenté aux figures **1A** et **1B** ;
- la figure **3** est une représentation schématique et vue de dessus du premier exemple de dispositif implantable selon l'invention représenté aux figures **1A, 1B** et **2** ;
- la figure **4** est une représentation schématique du premier exemple de dispositif selon l'invention représenté en fonctionnement disposé entre des vertèbres sus et sous-jacentes avec des portions dudit dispositif arrachées ;
- la figure **5** est une représentation schématique et en perspective d'un second exemple de dispositif selon l'invention ;
- la figure **6** est une représentation schématique et en perspective d'un premier exemple d'ensemble selon l'invention comprenant le deuxième exemple de dispositif implantable représenté à la figure **5** et des moyens de liaison postérieur des apophyses épineuses supérieure et inférieure entre-elles ;
- la figure **7** est une représentation schématique et en perspective d'un troisième exemple de dispositif implantable selon l'invention.

### Description détaillée des exemples de réalisation

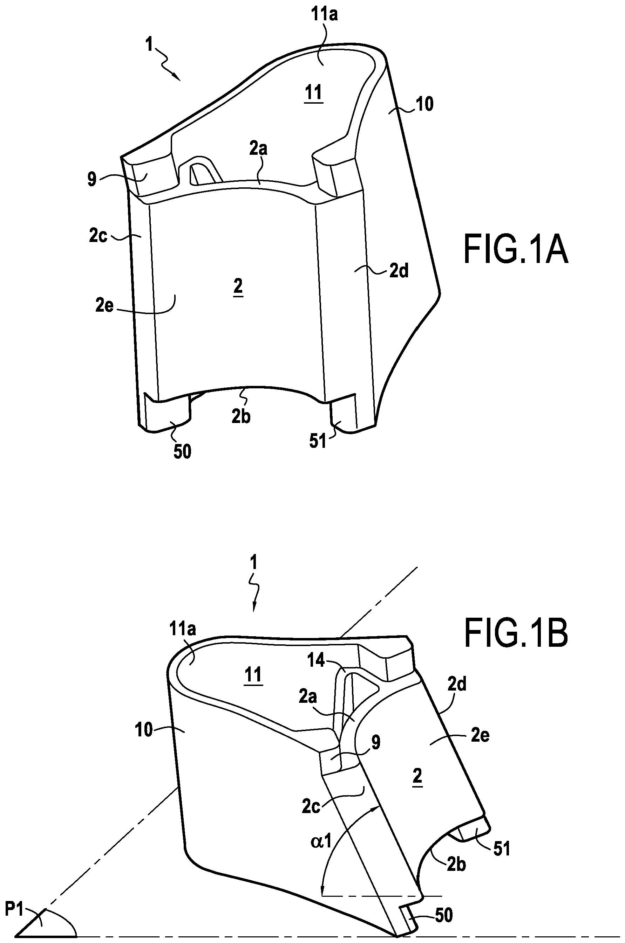

Le premier exemple de dispositif implantable d'arthrodèse vertébrale **1** pour la fusion de deux vertèbres sus et sous-jacentes représenté aux figures **1A, 1B**, **2, 3** **et** **4** comprend une partie antérieure **2** comprenant une zone supérieure d'appui **2a** apte à recevoir, au moins en partie, la partie inférieure **3** de la lame supérieure **4** de la vertèbre sus-jacente **5** et une zone inférieure d'appui **2b** apte à recevoir la partie supérieure **6** de la lame inférieure **7** de la vertèbre sous-jacente **8**, les zones supérieure **2a** et inférieure **2b** d'appui étant distantes d'une hauteur **h1** minimum pour le maintien d'un écart intervertébral. Le dispositif implantable **1** comprend également des moyens de retenue **9** rigides agencés par rapport à la partie antérieure **2** en sorte de bloquer la migration de ladite partie antérieure **2** vers le canal rachidien.

Le dispositif implantable **1** comprend aussi une partie postérieure **10**, en liaison avec ladite partie antérieure **2**, comprenant un logement principal **11** ayant des première **11a** et seconde **11b** ouvertures au regard l'une de l'autre et aptes à recevoir en partie les apophyses épineuses supérieure **12** et inférieure **13** respectivement des vertèbres sus-jacentes **5** et sous-jacentes **8.** Le logement principal **11** est rempli au moment de l'implantation d'un matériau d'ostéosynthèse granuleux (non représenté).

Dans cet exemple précis, les parties antérieure **2** et postérieure **10** sont rigides.

Le dispositif implantable **1** comprend un élément de cloisonnement **14** rigide disposé entre la partie antérieure **2** et la partie postérieure **10**, notamment dans un plan sensiblement parallèle au plan dans lequel la partie antérieure **2** est disposée.

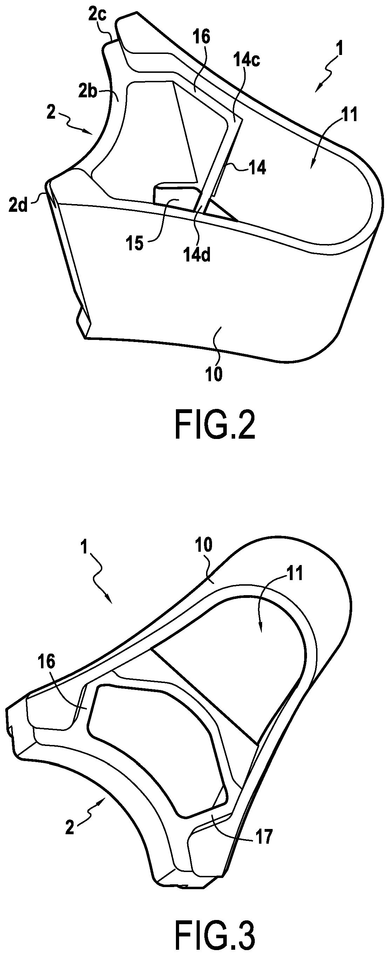

Dans cet exemple précis, les moyens de retenue **9** sont dans le prolongement de la partie postérieur **10**, à l'aplomb de la partie antérieure **2**, tel que cela est visible à la figure **2**.

L'élément de cloisonnement **14** délimite au moins en partie avec la partie antérieure **2** un logement auxiliaire **15** ayant des première **15a** et seconde **15b** ouvertures au regard l'une de l'autre, ledit logement auxiliaire **15** recevant en fonctionnement un matériau d'ostéosynthèse granuleux (non représenté), et agencé en sorte de recevoir en partie les lames **4,7** desdites vertèbres sus-jacente **5** et sous-jacente **8**.

La partie antérieure **2** comprend des premier **2c** et second **2d** bords latéraux en liaison avec les premier **14c** et second **14d** bords latéraux de l'élément de cloisonnement **14** respectivement par l'intermédiaire de première **16** et seconde **17** parties latérales. De préférence, les première **16** et seconde **17** parties latérales divergent vers la partie antérieure **2**.

L'élément de cloisonnement **14** a une forme générale de U, tel que cela est visible à la figure **3**. Dans cet exemple précis, la partie postérieure **10**, les moyens de retenue **9**, l'élément de cloisonnement **14** et la partie postérieure **2** sont dans une pièce moulée, par exemple en PEEK ou en hydroxyapatite.

La zone supérieure d'appui **2a** de la partie antérieure **2** a une forme convexe ; de préférence la zone inférieure d'appui **2b** de la partie antérieure **2** a une forme concave.

La partie antérieure **2** a une face antérieure **2e** concave. La partie antérieure **2** est inclinée vers ledit élément de cloisonnement **14**, en particulier forme un angle **α1** avec le plan d'appui (P1) sur lequel repose le dispositif implantable **1, α1** est inférieur à 90°, de préférence compris entre 45° et 90°.

La zone inférieure d'appui **2b** de la partie antérieure **2** comprend des premier **50** et second **51** pieds inclinés vers le logement principal **11.**

En fonctionnement, du matériau d'ostéosynthèse granuleux est disposé dans les logements auxiliaire **15** et principal **11,** de préférence en sorte de dépasser desdits logements **11,15** pour pallier au retrait de 20% en volume observé.

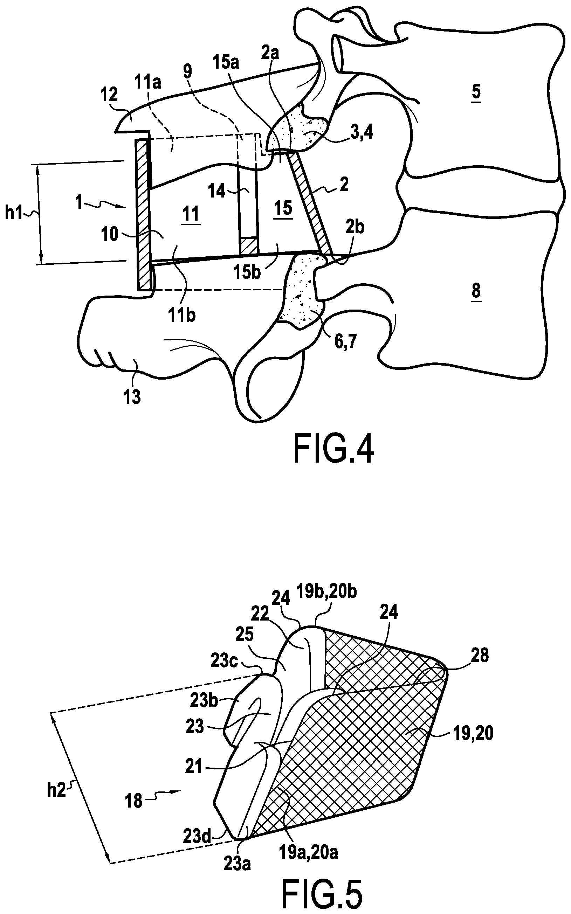

Le dispositif implantable **1** est ensuite disposé entre les lames **4,7** pour la partie antérieure **2** et entre les apophyses épineuses supérieure **12** et inférieure **13** pour la partie postérieure **10,** tel que représenté à la figure **4****.** Les apophyses épineuses **12,13** sont éventuellement entaillées, telles que représentées à la figure **4****,** en sorte de mieux pénétrer dans ledit logement principal **11** et de rentrer en contact avec le matériau d'ostéosynthèse.

La partie antérieure **2** permet de régler un écartement intervertébral. L'élément de cloisonnement **14** améliore la stabilité de la partie antérieure **2** et l'empêche de basculer vers les apophyses épineuses **12,13.** La surface de contact entre le matériau d'ostéosynthèse avec les lames supérieure **4** et inférieure **7** d'une part, et les apophyses épineuses supérieure **12** et inférieure **13** d'autre part, est importante, ce qui favorise une fusion osseuse au-delà des seules apophyses épineuses **12,13.**

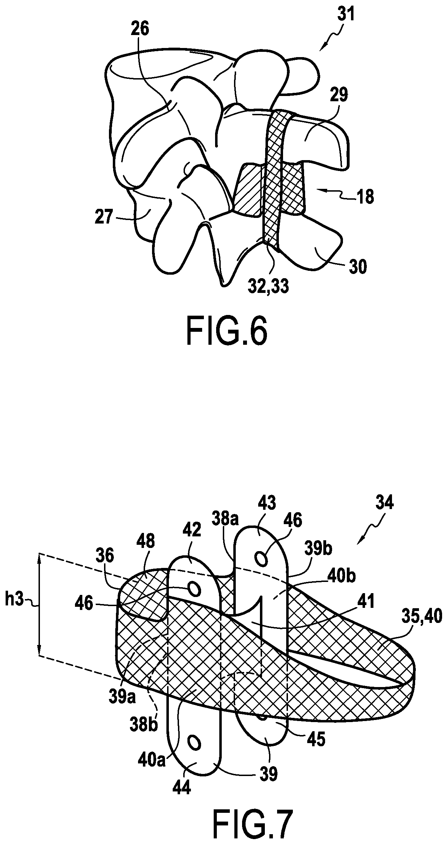

Le second exemple de dispositif implantable **18** représenté aux figures **5** et **6** ne sera décrit ci-après que de par ses différences avec le premier exemple de dispositif implantable **1.** La partie postérieure **19** est dans un matériau pliable, en particulier dans une bande pliable **20** dont la première extrémité **20a** et la seconde extrémité **20b** sont solidarisées respectivement avec les premier **25a** et second **25b** bords dudit élément de cloisonnement **25,** ainsi respectivement qu'aux première **21** et seconde **22** parties latérales reliant les premier **23a** et second **23b** bords latéraux de la partie antérieure **23** aux bords **25a** et **25b** dudit élément de cloisonnement **25.**

La partie antérieure **23** permet le maintien d'un écart vertébral d'une hauteur **h2** via les zones supérieure **23c** et inférieure **23d** d'appui.

Les moyens de retenue **24** sont ici supportés par l'élément de cloisonnement **25.** L'élément de cloisonnement **25** et les moyens de retenue **24** ont ainsi une forme générale en U. La partie antérieure **23,** l'élément de cloisonnement **25** et les moyens de retenue **24** sont rigides et dans cet exemple précis dans une seule pièce, par exemple en PEEK ou en hydroxyapatite moulée.

En fonctionnement, le second exemple de dispositif implantable **18** est disposé entre les vertèbres sus **26** et sous-jacentes **27** de la même manière que le premier exemple de dispositif implantable **1.** Le logement principal **28** étant dans un matériau pliable, tel que dans un panneau textile, permet aux apophyses épineuses **29,30** de rentrer davantage en contact avec le matériau d'ostéosynthèse lors des mouvements de lordose du rachis, ce qui favorise l'activation du greffon osseux et la formation de la fusion osseuse.

La figure **6** représente un premier exemple d'ensemble **31** selon l'invention comprenant le dispositif implantable **18** représenté à la figure **5****.** L'ensemble **31** comprend ainsi un moyen de solidarisation postérieur **32** des apophyses épineuses supérieure **29** et inférieure **30** entre-elles. Le moyen de solidarisation postérieure **32** comprend un élément longiligne **33** pourvu de première et seconde extrémités et d'un moyen de solidarisation desdites extrémités entre-elles. Ledit moyen de solidarisation postérieure **32** peut être amovible, tel que décrit dans FR 2.961.687 A1. Dans cet exemple précis, l'élément longiligne **32** entoure les apophyses épineuses supérieure **29** et inférieure **30** et la partie postérieure **19** comprenant un matériau d'ostéosynthèse granuleux. L'élément longiligne **33** contribue ainsi au maintien du matériau d'ostéosynthèse granuleux dans le logement principal **28,** en particulier lorsque le rachis est en extension.

Le troisième exemple de dispositif implantable **34** selon l'invention ne sera décrit ci-après que de par ses différences avec le premier exemple de dispositif implantable **1.** Le troisième exemple de dispositif implantable **34** comprend une partie postérieure **35** et une partie antérieure **36,** toutes deux dans un matériau pliable. La partie antérieure **36** est dans une bande pliable **38** dont les première **38a** et seconde **38b** extrémités sont solidarisées respectivement au premier bord **39a** et au second bord **39b** des moyens de retenue **39.** De même, la partie postérieure **35** est dans une bande pliable **40** dont les première **40a** et seconde **40b** extrémités sont solidarisées respectivement au premier bord **39a** et au second bord **39b** des moyens de retenue **39.**

L'élément de cloisonnement **41** et les moyens de retenue **39** ont ensemble une forme générale de H. Les moyens de retenue **39** comprennent des première **42** et seconde **43** projections supérieures et des première **44** et seconde **45** projections inférieures s'étendant respectivement des premier **39a** et second **39b** bords latéraux desdits moyens de retenue **39.** Ces projections **42** à **45** sont pourvues d'au moins un orifice **46** apte à coopérer avec des vis (non représentées). Lesdites projections **41** à **45** et les vis forment ainsi un moyen de solidarisation postérieure **47** du dispositif implantable **34** aux apophyses épineuses supérieure et inférieure.

La disposition d'un matériau d'ostéosynthèse de façon suffisamment compactée dans le logement auxiliaire **48** délimité entre la partie antérieure **36** et l'élément de cloisonnement **41** permet de former des zones supérieure **35a** et inférieure **35b** d'appui aptes à venir en contact avec les lames supérieure et inférieure et ainsi maintenir un écartement vertébral minimum, correspondant à la hauteur minimale **h2** de la partie antérieure **36.**

La déformation au moins en partie du logement auxiliaire **48** permet d'améliorer la fusion osseuse car le matériau d'ostéosynthèse est davantage stimulé par les lames des vertèbres sus et sous-jacentes du niveau vertébral à corriger.

## Revendications

1. Dispositif implantable d'arthrodèse vertébrale (1,18,34) pour la fusion de deux vertèbres sus et sous-jacentes, comprenant :
a. une partie antérieure (2,23,36) comprenant une zone supérieure d'appui (2a,23c) apte à recevoir la partie inférieure (3) de la lame supérieure (4) de la vertèbre sus-jacente (5) et une zone inférieure d'appui (2b,23d) apte à recevoir la partie supérieure (6) de la lame inférieure (7) de la vertèbre sous-jacente (8), les zones supérieure (2a) et inférieure (2b) d'appui étant distantes d'une hauteur h minimum (h1,h2,h3) pour le maintien d'un écart intervertébral ;
b. des moyens de retenue (9) rigides agencés par rapport à la partie antérieure (2) en sorte de bloquer la migration de ladite partie antérieure (2) vers le canal rachidien ;
c. une partie postérieure (10,19,35), en liaison avec ladite partie antérieure (2), comprenant un logement principal (11,28) ayant des première (11a) et seconde (11b) ouvertures au regard l'une de l'autre et aptes à recevoir en partie les apophyses épineuses (12,13,29,30) respectivement des vertèbres sus-jacente (5,26) et sous-jacente (8,27) ;
**caractérisé en ce qu'**il comprend :
d. un élément de cloisonnement (14,25,41) rigide disposé entre la partie antérieure (2) et la partie postérieure (10) qui délimite, au moins en partie avec la partie antérieure (2), un logement auxiliaire (15) ayant des première (15a) et seconde (15b) ouvertures au regard l'une de l'autre, et agencé en sorte de recevoir en partie les lames (4,7) desdites vertèbres sus-jacente (5) et sous-jacente (8) ;
e. un matériau d'ostéosynthèse granuleux disposé dans ledit logement principal (11) et ledit logement auxiliaire (15).

2. Dispositif implantable (18,34) selon la revendication **1, caractérisé en ce que** le logement principal (28) est dans un matériau pliable, de préférence dans une bande pliable (20,40) dont la première extrémité (20a,40a)et la seconde extrémité (20b,40b) sont solidarisées respectivement au premier bord latéral (25a,39a) et au second bord latéral (25b,39b) de ladite partie antérieure et/ou desdits moyens de retenue (39) et/ou dudit élément de cloisonnement (25).

3. Dispositif implantable (18) selon l'une ou l'autre des revendications **1** et **2, caractérisé en ce que** les moyens de retenue (24) sont supportés par ledit élément de cloisonnement (25) ou ladite partie postérieure.

4. Dispositif implantable (1) selon l'une quelconque des revendications **1** à **3, caractérisé en ce que** la partie antérieure (2) comprend des premier (2c) et second (2d) bords latéraux en liaison avec les premier (14c) et second (14d) bords latéraux de l'élément de cloisonnement (14) respectivement par l'intermédiaire de première (16) et seconde (17) parties latérales.

5. Dispositif implantable (1) selon la revendication **4, caractérisé en ce que** les première (16) et seconde (17) parties latérales divergent vers la partie antérieure (2).

6. Dispositif implantable (1,18,34) selon l'une quelconque des revendications **1** à **5, caractérisé en ce que** l'élément de cloisonnement (14,25,41), et éventuellement les moyens de retenue (24,39), a ou ont une forme générale de U ou de H.

7. Dispositif implantable (1) selon l'une quelconque des revendications **1** à **6, caractérisé en ce que** la zone supérieure (2a) d'appui de la partie antérieure (2) a une forme convexe, de préférence la zone inférieure (2b) d'appui de la partie antérieure (2) a une forme concave.

8. Dispositif implantable (1) selon l'une quelconque des revendications **1** à **7, caractérisé en ce que** la partie antérieure (2) a une face antérieure (2c) concave.

9. Dispositif implantable (1) selon l'une quelconque des revendications **1** à **8, caractérisé en ce que** la partie antérieure (2) est inclinée vers ledit élément de cloisonnement (14).

10. Dispositif implantable (1) selon l'une quelconque des revendications **1** à **9, caractérisé en ce que** la zone inférieure d'appui (2b) de la partie antérieure (2) comprend des premier (50) et second (51) pieds inclinés vers le logement principal (11).

11. Dispositif implantable (34) selon l'une quelconque des revendications **1** à **10, caractérisé en ce que** les moyens de retenue (39) comprennent au moins une première projection supérieure (42,43) et au moins une première projection inférieure (44,45), qui s'étendent du premier (39a) et/ou du second (39b) bord(s) latéra(l)(ux) dudit élément de cloisonnement, et/ou dudit moyen de retenue (39), et sont pourvus à leurs extrémités d'orifices (46) pour le passage de vis.

12. Dispositif implantable (18) selon l'une quelconque des revendications **1** à **11, caractérisé en ce que** la partie antérieure (23), les moyens de retenue (24), éventuellement la partie postérieure et/ou l'élément de cloisonnement (25), sont dans un matériau d'ostéosynthèse.

13. Ensemble (31) comprenant un dispositif implantable (18) selon l'une quelconque des revendications **1** à **12,** et un moyen de solidarisation postérieur (32) des apophyses épineuses supérieure (29) et inférieure (30) entre-elles, de préférence ledit moyen de solidarisation postérieur (32) comprend un élément longiligne (33) pourvu de première et seconde extrémités, et d'un moyen de solidarisation desdites extrémités entre elles.

## Patentansprüche

1. Implantierbare Vorrichtung zur vertebralen Arthrodese (1, 18, 34) für die Fusion von zwei darüber- und darunterliegenden Wirbeln, umfassend:
a. einen vorderen Teil (2, 23, 36) mit einem oberen Anlagebereich (2a, 23c), der geeignet ist, den unteren Teil (3) der oberen Platte (4) des darüberliegenden Wirbels (5) aufzunehmen, sowie einem unteren Anlagebereich (2b, 23d), der geeignet ist, den oberen Teil (6) der unteren Platte (7) des darunterliegenden Wirbels (8) aufzunehmen, wobei der obere (2a) und der untere (2b) Anlagebereich um eine Mindesthöhe h (h1, h2, h3) für das Aufrechterhalten eines Zwischenwirbelabstandes entfernt sind,
b. starre Haltemittel (9), die gegenüber dem vorderen Teil (2) angeordnet sind, um das Wandern des vorderen Teils (2) in Richtung des Wirbelkanals zu blockieren,
c. einen hinteren Teil (10, 19, 35), in Verbindung mit dem vorderen Teil (2), der eine Hauptaufnahme (11, 28) mit einer ersten (11a) und einer zweiten (11b) Öffnung umfasst, welche einander gegenüberliegen und geeignet sind, die Dornfortsätze (12, 13, 29, 30) des darüberliegenden Wirbels (5, 26) bzw. des darunterliegenden Wirbels (8, 27) teilweise aufzunehmen,
**dadurch gekennzeichnet, dass** sie umfasst:
d. ein zwischen dem vorderen Teil (2) und dem hinteren Teil (10) angeordnetes starres Unterteilungselement (14, 25, 41), das wenigstens teilweise mit dem vorderen Teil (2) eine Hilfsaufnahme (15) begrenzt, welche einander gegenüberliegende erste (15a) und zweite (15b) Öffnungen aufweist und derart angeordnet ist, dass sie die Platten (4, 7) der darüberliegenden (5) und darunterliegenden (8) Wirbel teilweise aufnimmt,
e. ein körniges Osteosynthesematerial, das in der Hauptaufnahme (11) und der Hilfsaufnahme (15) angeordnet ist.

2. Implantierbare Vorrichtung (18, 34) nach Anspruch 1, **dadurch gekennzeichnet, dass** die Hauptaufnahme (28) aus einem biegsamen Material, vorzugsweise aus einem biegsamen Band (20, 40) besteht, dessen erstes Ende (20a, 40a) und zweites Ende (20b, 40b) mit dem ersten Seitenrand (25a, 39a) bzw. mit dem zweiten Seitenrand (25b, 39b) des vorderen Teils und/oder der Haltemittel (39) und/oder des Unterteilungselements (25) fest verbunden sind.

3. Implantierbare Vorrichtung (18) nach dem einen oder dem anderen der Ansprüche 1 und 2, **dadurch gekennzeichnet, dass** die Haltemittel (24) von dem Unterteilungselement (25) oder dem hinteren Teil getragen sind.

4. Implantierbare Vorrichtung (1) nach einem der Ansprüche 1 bis 3, **dadurch gekennzeichnet, dass** der vordere Teil (2) erste (2c) und zweite (2d) Seitenränder in Verbindung mit den ersten (14c) und zweiten (14d) Seitenrändern des Unterteilungselements (14) mittels eines ersten (16) bzw. eines zweiten (17) Seitenteils umfasst.

5. Implantierbare Vorrichtung (1) nach Anspruch 4, **dadurch gekennzeichnet, dass** der erste (16) und der zweite (17) Seitenteil in Richtung des vorderen Teils (2) auseinanderlaufen.

6. Implantierbare Vorrichtung (1, 18, 34) nach einem der Ansprüche 1 bis 5, **dadurch gekennzeichnet, dass** das Unterteilungselement (14, 25, 41) und eventuell die Haltemittel (24, 39) insgesamt U- oder H-förmig ausgebildet ist oder sind.

7. Implantierbare Vorrichtung (1) nach einem der Ansprüche 1 bis 6, **dadurch gekennzeichnet, dass** der obere Anlagebereich (2a) des vorderen Teils (2) eine konvexe Form aufweist, vorzugsweise der untere Anlagebereich (2b) des vorderen Teils (2) eine konkave Form aufweist.

8. Implantierbare Vorrichtung (1) nach einem der Ansprüche 1 bis 7, **dadurch gekennzeichnet, dass** der vordere Teil (2) eine konkave Vorderseite (2c) aufweist.

9. Implantierbare Vorrichtung (1) nach einem der Ansprüche 1 bis 8, **dadurch gekennzeichnet, dass** der vordere Teil (2) in Richtung des Unterteilungselements (14) geneigt ist.

10. Implantierbare Vorrichtung (1) nach einem der Ansprüche 1 bis 9, **dadurch gekennzeichnet, dass** der untere Anlagebereich (2b) des vorderen Teils (2) einen ersten (50) und einen zweiten (51) Fuß, die in Richtung der Hauptaufnahme (11) geneigt sind, umfasst.

11. Implantierbare Vorrichtung (34) nach einem der Ansprüche 1 bis 10, **dadurch gekennzeichnet, dass** die Haltemittel (39) wenigstens einen ersten oberen Vorsprung (42, 43) und wenigstens einen ersten unteren Vorsprung (44, 45) umfassen, die sich von dem ersten (39a) und/oder von dem zweiten (39b) Seitenrand (Seitenrändern) des Unterteilungselements, und/oder des Halteelements (39), erstrecken und an ihren Enden mit Öffnungen (46) für den Durchgang von Schrauben versehen sind.

12. Implantierbare Vorrichtung (18) nach einem der Ansprüche 1 bis 11, **dadurch gekennzeichnet, dass** der vordere Teil (23), die Haltemittel (24), eventuell der hintere Teil und/oder das Unterteilungselement (25), aus einem Osteosynthesematerial bestehen.

13. Anordnung (31), umfassend eine implantierbare Vorrichtung (18) nach einem der Ansprüche 1 bis 12 und ein hinteres Mittel zum festen Verbinden (32) des oberen (29) und des unteren (30) Dornfortsatzes untereinander, vorzugsweise umfasst das hintere Mittel zum festen Verbinden (32) ein langgestrecktes Element (33), das mit einem ersten und einem zweiten Ende und mit einem Mittel zum festen Verbinden der Enden untereinander versehen ist.

## Claims

1. An implantable vertebral arthrodesis device (1, 18, 34) for fusing together an underlying vertebra and an overlying vertebra comprising:
a) an anterior portion (2, 23, 36) having an upper bearing zone (2a, 23c) suitable for receiving the lower portion (3) of the upper lamina (4) of the overlying vertebra (5) and a lower bearing zone (2b, 23d) suitable for receiving the upper portion (6) of the lower lamina (7) of the underlying vertebra (8), the upper and lower bearing zones (2a and 2b) being spaced apart by a minimum height h (h1, h2, h3), in order to maintain an intervertebral spacing;
b) rigid retaining devices (9) arranged relative to the anterior portion (2) in such a manner as to block migration of said anterior portion (2) towards the spinal canal;
c) a posterior portion (10, 19, 35) in connection with said anterior portion (2), comprising a main housing (11, 28) having first and second openings (11a and 11b) facing each other and suitable for receiving in part the spinous processes (12, 13, 29, 30) respectively of the underlying and overlying vertebrae (8, 27 and 5, 26);
**characterized in that** it comprises:
d) a rigid partitioning element (14, 25, 41) arranged between the anterior portion (2) and the posterior portion (10) that co-operates, at least in part with the anterior portion (2), to define an auxiliary housing (15) having first and second openings (15a and 15b) facing each other and arranged in such a manner as to receive in part the laminae (4, 7) of said underlying and overlying vertebrae (8 and 5); and
e) a granular osteosynthesis material arranged in said main housing (11) and said auxiliary housing (15).

2. An implantable device (18, 34) according to claim 1, **characterized in that** the main housing (28) is made of a flexible material, preferably a flexible strip (20, 40) having a first end (20a, 40a) and a second end (20b, 40b) that are secured respectively to the first lateral edge (25a, 39a) and to the second lateral edge (25b, 39b) of said anterior portion and/or of said retaining devices (39) and/or of said partitioning element (25).

3. An implantable device (18) according to claim 1 or claim 2, **characterized in that** the retaining devices (24) are supported by said partitioning element (25) or said posterior portion.

4. An implantable device (1) according to any one of claims 1 to 3, **characterized in that** the anterior portion (2) comprises first and second lateral edges (2c and 2d) in connection with the first and second lateral edges (14c and 14d) of the partitioning element (14) respectively via first and second lateral portions (16 and 17).

5. An implantable device (1) according to claim 4, **characterized in that** the first and second lateral portions (16 and 17) diverge towards the anterior portion (2).

6. An implantable device (1, 18, 34) according to any one of claims 1 to 5, **characterized in that** the partitioning element (14, 25, 41) and possibly the retaining devices (24, 39) is/are generally U-shaped or H-shaped.

7. An implantable device (1) according to any one of claims 1 to 6, **characterized in that** the upper bearing zone (2a) of the anterior portion (2) is convex in shape, and the lower bearing zone (2b) of the anterior portion (2) is preferably concave in shape.

8. An implantable device (1) according to any one of claims 1 to 7, **characterized in that** the anterior portion (2) has an anterior face (2c) that is concave.

9. An implantable device (1) according to any one of claims 1 to 8, **characterized in that** the anterior portion (2) slopes towards said partitioning element (14).

10. An implantable device (1) according to any one of claims 1 to 9, **characterized in that** the lower bearing zone (2b) of the anterior portion (2) comprises first and second feet (50 and 51) sloping towards the main housing (11).

11. An implantable device (34) according to any one of claims 1 to 10, **characterized in that** the retaining devices (39) comprise at least one first upper projection (42, 43) and at least one first lower projection (44, 45), which projections extend from the first and/or second lateral edge(s) (39a and/or 39b) of said partitioning element, and/or of said retaining device (39), and are provided at their ends with respective orifices (46) for passing screws.

12. An implantable device (18) according to any one of claims 1 to 11, **characterized in that** the anterior portion (23), the retaining devices (24), and optionally the posterior portion and/or the partitioning element (25) are made of osteosynthesis material.

13. A set (31) comprising an implantable device (18) according to any one of claims 1 to 12, and posterior securing device (32) for securing together the upper and lower spinous processes (29 and 30), said posterior securing device (32) preferably comprising an elongate element (33) having first and second ends, and a securing device for securing said ends together.
